# EUROPEAN PATENT SPECIFICATION

(11) **EP 3 777 762 B1**
(45) Date of publication and mention of the grant of the patent: **27.11.2024**
(21) Application number: 19784598.5
(22) Date of filing: 29.03.2019
(51) Int. Cl.: A61C 17/02, A61C 17/024, A61B 1/24, A61B 1/04

(54) **DENTAL IRRIGATOR COMPRISING WATER QUANTITY STEPLESS REGULATION DEVICE**
ZAHNSPÜLVORRICHTUNG MIT STUFENLOSEM WASSERMENGENREGLER
IRRIGATEUR DENTAIRE COMPRENANT UN DISPOSITIF DE RÉGULATION EN CONTINU DE QUANTITÉ D'EAU

(30) Priority: 13.04.2018 CN 201810332798
(43) Date of publication of application: 17.02.2021
(73) Proprietor: Zhou, Xing, Guangdong 510663 (CN)
(72) Inventor: Zhou, Xing, Guangdong 510663 (CN)
(74) Representative: Boult Wade Tennant LLP
(86) International application number: PCT/CN2019/080386
(87) International publication number: WO 2019/196678

(56) References cited:
- EP-A1- 2 541 214
- CN-A- 101 716 099
- CN-A- 107 789 083
- CN-A- 107 789 083
- CN-U- 204 766 005
- CN-U- 204 766 005
- KR-B1- 101 740 507
- US-A1- 2014 127 641
- ANONYMOUS: "Potentiometer - Wikipedia", 30 December 2016 (2016-12-30), XP055342078, Retrieved from the Internet <URL:https://en.wikipedia.org/wiki/Potentiometer> [retrieved on 20170203]

## Description

### FIELD OF THE INVENTION

The invention relates to a tool for oral cleaning, and particularly relates to an oral irrigator with a water volume stepless adjustment device for cleaning teeth, interdental spaces and oral cavities.

### BACKGROUND OF THE INVENTION

Due to reasons of age, pathology, and the like, gingival atrophy and tooth gap expansion are prone to occur, which is easy to cause food residues. If the food residues cannot be cleaned in time, on the one hand, it will cause bad breath, and on the other hand, it is easy to cause various dental diseases, oral diseases and especially periodontitis.

In view of this situation, various dental flosses, interdental brushes, water flosses and other products have been developed on the market for users to choose. Particularly, due to convenient use and good cleaning effect, water floss products are oral cleaning products widely used on the current market.

The working principle of the water floss is to clean the teeth and the oral cavity by the impact force of a high-speed water column sprayed under a certain pressure. Therefore, the high-speed water column will inevitably generate a very large impact on the teeth, gingivae and the like in the oral cavity. However, according to their own oral conditions, the tolerance of each person to the impact force of the water column is different. Therefore, in the using process, each person needs to select different water column impact speeds according to their tolerance. The current water floss generally adopts a grading treatment method which allows the user to select different gears. Although this method is simple in operation, it is often impossible to select the most suitable water column impact force which considers both the cleaning effect and comfort.

In order to overcome the defects of the prior art and to enable the user to select the most comfortable water column impact force, it is necessary to develop an oral irrigator capable of continuously adjusting the water volume.

EP 2541214 A1 describes a personal care device, such as an oral care device, provided with a controller and adjusting means for inputting a desired value and/or desired variation for an operating parameter. The adjusting means include at least one variable input element, such as a potentiometer or a contactless encoder connectable to the controller.

US 2014/127641 A1 describes an oral irrigator that has a pump unit having at least a first pump being connected or being connectable to at least a first fluid reservoir containing a first fluid, a control unit for controlling the pump unit, at least a first outlet nozzle for emitting fluid pumped from the first fluid reservoir to the first outlet nozzle during operation as a continuous or pulsating fluid jet, wherein the control unit is arranged to control the pump unit during operation in at least a first operation mode such that at least a first fluid jet parameter of the continuous or pulsating jet is automatically varied over time.

CN 204766005 U describes a dental irrigator comprising a rinsing head, a pump body assembly in communication with the rinsing head, a hose in communication with the pump body assembly, and a water tank in communication with the hose; the pump body assembly includes a pump, which is electrically connected to a motor and a battery is electrically connected to the motor; the pump body assembly and the hose are located in a casing, the rinsing head and the water tank are located outside the casing, and a switch adjustment button is provided on the casing, The switch adjustment button is connected with the pump body assembly.

CN 107789083 A describes a water toothpick assembly and auxiliary equipment thereof. One end of a water toothpick is equipped with a spray nozzle, and the other end of the water toothpick is equipped with a spray nozzle base; the spray nozzle is connected with the spray nozzle base through a blocking part; the auxiliary equipment comprises a housing, a power supply and a water pump; the housing comprises a water receiving part which is positioned on the side surface of the shell and is used for connecting a water tube outside the shell; the water pump is placed in the shell for producing continuous high-pressure outlet water; and the power supply is fixedly connected with the water pump for charging the water pump. A water inlet is connected with the water pump through an internal water tube, and the water pump generates continuous high-pressure outlet water.

### SUMMARY

The present invention provides an oral irrigator as set out in claim 1.

In an oral irrigator with a water volume stepless adjustment device of the invention, the water volume stepless adjustment device is disposed on the oral irrigator to realize stepless continuous adjustment of the water volume, so that a user can select the most comfortable water volume and water column impact force according to the own feeling in a using process.

The oral irrigator with a water volume stepless adjustment device of the invention is characterized in that: an oral irrigator 100 with a water volume stepless adjustment device includes a sprayer 1, a spray lance 2, a control switch 3, a vessel 4, a water pipe 5 and a water volume stepless adjustment device 6;

A. the sprayer 1 is disposed at a front end of the spray lance 2; the control switch 3 is capable of controlling the spraying of a fluid in the spray lance 2; a fluid in the vessel 4 is connected to the spray lance 2 through the water pipe 5;

B. the water volume stepless adjustment device 6 includes an adjusting switch 61, a controller 62 and a signal output device 63; a signal output by the controller 62 can be continuously adjusted by adjusting the adjusting switch 61 so as to continuously adjust the magnitude of pressure of the fluid sprayed from the spray lance 2.

Because the adjusting switch 61 of the water volume stepless adjustment device 6 can continuously adjust the signal output by the controller 62, the signal output by the output device 63 may be continuously changed, and the magnitude of the pressure of the fluid sprayed from the spray lance 2 can be continuously adjusted by continuously adjusting the output signal of the controller 62. When a user uses the oral irrigator, the user can adjust water pressure according to personal tolerance and preferences until feel most comfortable, thereby greatly enhancing the cleaning effect and comfort of the oral irrigator in the using process.

The signal adjusted by the controller 62 is voltage, current, power, frequency, or waveform. Herein, the applicant only enumerates the above signals, but those skilled in the art can adjust other signals according to needs so as to adjust the magnitude of the pressure of the fluid without departing from the scope of protection of the present application.

The adjusting switch 61 is a knob type adjusting switch.

The adjusting switch 61 includes a knob 61-1 and a positioning hole 61-2. The controller 62 includes a potentiometer 62-1 and a control chip 62-2, an output signal of the potentiometer 62-1 is an input signal of the control chip 62-2, and an output signal of the control chip 62-2 can be controlled. The potentiometer 62-1 is provided with a connecting rod 62-1-1, and the connecting rod 62-1-1 can be embedded in the positioning hole 61-2. The knob 61-1 can be rotated to drive the connecting rod 62-1-1 to rotate to adjust the magnitude of the output signal of the potentiometer 62-1, thereby adjusting the output signal of the control chip 62-2.

The knob type adjusting switch can continuously adjust the output signal, such as a voltage signal, of the potentiometer 62-1 by rotating the knob 61-1, the output signal, such as the voltage signal, of the potentiometer 62-1 is the input signal of the control chip 62-2, and the output signal, such as the magnitude of the output voltage, the magnitude of the current, the magnitude of the power, the magnitude of the frequency or the pulse waveform, of the control chip 62-2 can be continuously adjusted by adjusting the input signal of the control chip 62-2. The signal output by the control chip 62-2 can control the magnitude of pressure of water sucked by the water suction device 7 or the magnitude of pressure applied by the pressurizing device 41-1 to the fluid in the pressure vessel 41. The pressure of the fluid sprayed from the sprayer 1 can be continuously adjusted by continuously adjusting the signal output by the control chip 62-2. The knob type adjusting switch only needs to rotate the knob 61-1 back and forth to realize signal adjustment of the controller 62, so that the use is simple and convenient.

In an example outside the scope of the present invention, but included as useful for understanding the invention, the adjusting switch 61 is a slide groove type adjusting switch.

The adjusting switch 61 according to the example outside the scope of the present invention includes a handle 61-3 and a slide block 61-4. The potentiometer 62-1 is provided with a slide groove 62-1-2. The slide block 61-4 is embedded in the slide groove 62-1-2. The handle 61-3 is pushed back and forth to enable the slide block 61-4 to move back and forth in the slide groove 62-1-2 to adjust the magnitude of the output signal of the potentiometer 62-1, thereby adjusting the output signal of the control chip 62-2.

By embedding the slide block 61-4 in the slide groove 62-1-2, the slide groove type adjusting switch only needs to push the handle 61-3 back and forth to realize signal adjustment of the controller 62, so that the operation is very convenient.

Herein, the applicant enumerates a knob type adjusting mode according to the scope of protection of the present invention. The slide groove type adjusting mode is provided as an example outside the scope of the present invention.

The oral irrigator 100 includes a water suction device 7. The water suction device 7 is capable of sucking the fluid in the vessel 4 into the spray lance 2 through the water pipe 5, and then, the fluid is sprayed from the sprayer 1.

The water suction device 7 is a manual water suction device or an electric water suction device 71.

Further, the water suction device 7 is an electric water suction device 71. The electric water suction device 71 includes a water inlet pipe 71-1, a motor 71-2 and a water outlet pipe 71-3. The motor 71-2 works, the fluid in the vessel 4 is sucked into the electric water suction device 71 through the water pipe 5 and then sprayed from the water outlet pipe 71-3, and the sprayed fluid passes through the water pipe 5 and is then sprayed from the spray lance 2.

The oral irrigator with a water volume stepless adjustment device of the invention also includes a power source 8 and a circuit system 9. The power source 8, the electric water suction device 71, the water volume stepless adjustment device 6 and the control switch 3 are connected together through the circuit system 9.

After the output signal of the controller 62 of the water volume stepless adjustment device 6 is output by the signal output device 63, a rotation speed of the motor 71-2 can be controlled, and the rotation speed of the motor 71-2 can be adjusted by adjusting the output signal of the controller 62, thereby adjusting the magnitude of the pressure of the fluid sprayed from the sprayer 1.

The vessel 4 is detachably mounted at a proximal end of the oral irrigator 100. By virtue of a detachable connection mode, the vessel 4 can be conveniently detached and then cleaned, so that the vessel 4 is more sanitary and safe for long-term use.

During use, the control switch 3 is turned on, the motor 71-2 of the electric water suction device 71 works, the fluid in the vessel 4 is sucked into the electric water suction device 71 through the water pipe 5 and then sprayed from the water outlet pipe 71-3, and the sprayed fluid enters the spray lance 2 through the water pipe 5 and is finally sprayed from the sprayer 1 of the spray lance 2. The knob 61-1 is rotated or the handle 61-3 is pushed back and forth to continuously adjust the magnitude of the pressure of the sprayed fluid, an appropriate water pressure is selected according to personal preferences and cleaning effects, and then, the sprayer 1 is aligned to a part to be cleaned so as to clean the teeth and the oral cavity.

The vessel 4 is a pressure vessel 41. The pressure vessel 41 includes a pressurizing device 41-1, a housing 41-2 and a fluid containing space 41-3. The pressurizing device 41-1 capable of increasing the pressure of the fluid is mounted on the housing 41-2.

The vessel 4 may also be a pressure vessel 41. The pressurizing device 41-1 of the pressure vessel 41 may be an electric pressurizing device 41-11. The electric pressurizing device 41-11 can increase the pressure of a fluid in the fluid containing space 41-3, so that the fluid in the fluid containing space 41-3 is pressed into the water pipe 5 from the fluid containing space 41-3 under the action of the external pressure, and then is sprayed from the sprayer of the spray lance 2. The controller 62 of the water volume stepless adjustment device 6 can control the magnitude of the pressure applied by the electric pressurizing device 41-11 to the fluid in the fluid containing space 41-3 through the signal output by the output device 63, and the magnitude of the pressure of the fluid sprayed from the spray lance 2 can be controlled by continuously adjusting the magnitude of the applied pressure.

During use, the control switch 3 is turned on, the electric pressurizing device 41-11 works, the fluid in the fluid containing space 41-3 is pressurized, and under the action of the pressure, the fluid in the fluid containing space 41-3 is pressed into the spray lance 2 through the water pipe 5 and is finally sprayed from the sprayer. The knob 61-1 is rotated or the handle 61-3 is pushed back and forth to adjust the magnitude of the pressure of the sprayed fluid, an appropriate water pressure is selected according to personal preferences and cleaning effects, and then, the sprayer 1 is aligned to the part to be cleaned so as to clean the teeth and the oral cavity.

The oral irrigator 100 can be connected to the oral observation instrument 200 through the connecting mechanism 300. The oral irrigator of the invention can also be connected to the oral observation instrument 200 through the connecting mechanism 300, and the sprayer 1 of the spray lance 2 is within the field of view of the observation system 24 of the oral observation instrument 200, so that under direct vision, the water volume is adjusted, the teeth and the oral cavity are cleaned, and the cleaning process can be shot and photographed.

The oral irrigator with a water volume stepless adjustment device of the invention includes a sprayer 1, a spray lance 2, a control switch 3, a vessel 4, a water pipe 5 and a water volume stepless adjustment device 6. The water volume stepless adjustment device 6 includes an adjusting switch 61, a controller 62 and a signal output device 63. The signal output by the controller 62 can be continuously adjusted by adjusting the adjusting switch 61 so as to continuously adjust the magnitude of the pressure of the fluid sprayed from the spray lance 2. When a user uses the oral irrigator with a water volume stepless adjustment device of the invention, the user can adjust the water pressure according to personal tolerance and preferences until feel most comfortable, thereby greatly enhancing the cleaning effect and comfort of the oral irrigator in the using process. The oral irrigator with a water volume stepless adjustment device of the invention can also be connected to the oral observation instrument 200, so that under direct vision, the water volume is adjusted, the teeth and the oral cavity are cleaned, and the cleaning process can be shot and photographed.

### BRIEF DESCRIPTION OF THE DRAWINGS

FIG. 1 is a schematic perspective view of a knob adjusting type oral irrigator with a water volume stepless adjustment device of the invention.
FIG. 2 is a front view of FIG. 1.
FIG. 3 is a rear view of FIG. 1.
FIG. 4 is a partial cross-sectional view of FIG. 1.
FIG. 5 is a top view of FIG. 1.
FIG. 5-1 is a cross-sectional view along A-A of FIG. 5.
FIG. 6 is an exploded view of FIG. 1.
FIG. 7 is a schematic perspective view of a slide block adjusting type oral irrigator with a water volume stepless adjustment device according to an example outside the scope of the present invention.
FIG. 8 is a front view of FIG. 7.
FIG. 9 is a rear view of FIG. 7.
FIG. 10 is a partial cross-sectional view of FIG. 7.
FIG. 11 is a cross-sectional view of FIG. 1.
FIG. 12 is an exploded view of FIG. 7.
FIG. 13 is a schematic view of an oral irrigator with a water volume stepless adjustment device of the invention, where the oral irrigator includes a pressure vessel.
FIG. 14 is a schematic view of an oral irrigator with a water volume stepless adjustment device of the invention, where the oral irrigator is capable of being connected to an oral observation instrument.

In the above drawings:
- oral irrigator 100 with water volume stepless adjustment device of the invention, oral observation instrument 200, connecting mechanism 300;
- sprayer 1, spray lance 2, control switch 3, vessel 4, water pipe 5, water volume stepless adjustment device 6, water suction device 7, power source 8, circuit system 9;
- pressure vessel 41, pressurizing device 41-1, electric pressurizing device 41-11, air inlet 41-11-1, compressor 41-11-2, air outlet 41-11-3, housing 41-2, fluid containing space 41-3;
- adjusting switch 61, knob 61-1, positioning hole 61-2, handle 61-3, slide block 61-4, controller 62, potentiometer 62-1, control chip 62-2, signal output system 62-3, connecting rod 62-1-1, slide groove 62-1-2;
- electric water suction device 71, water inlet pipe 71-1, motor 71-2, water outlet pipe 71-3; and
- observation system 24 of oral observation instrument.

### DESCRIPTION OF EMBODIMENTS

### Embodiment 1: knob adjusting type oral irrigator with water volume stepless adjustment device of the invention

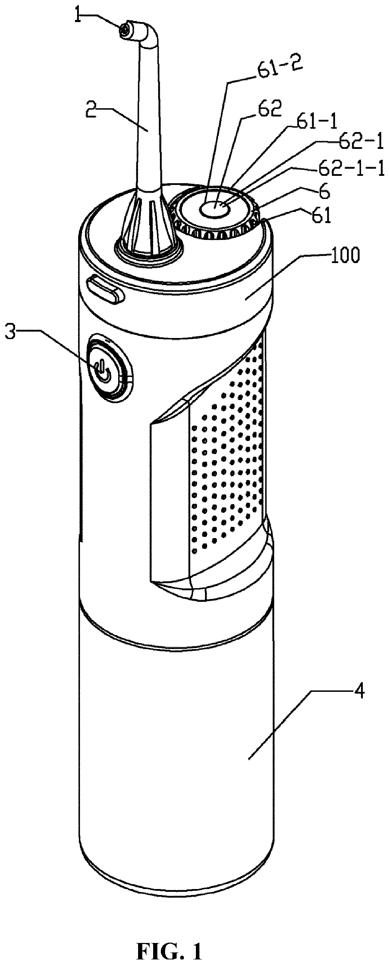

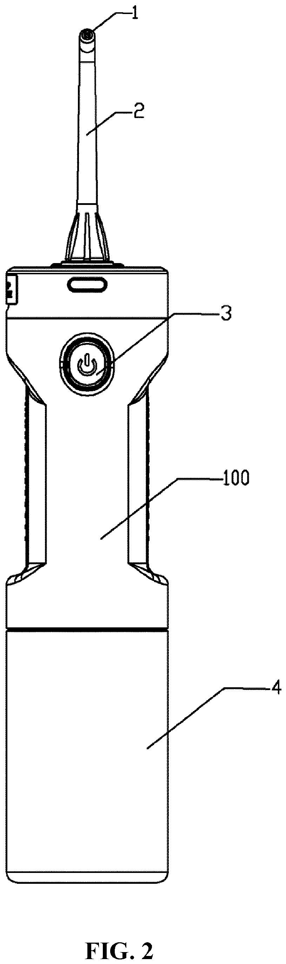

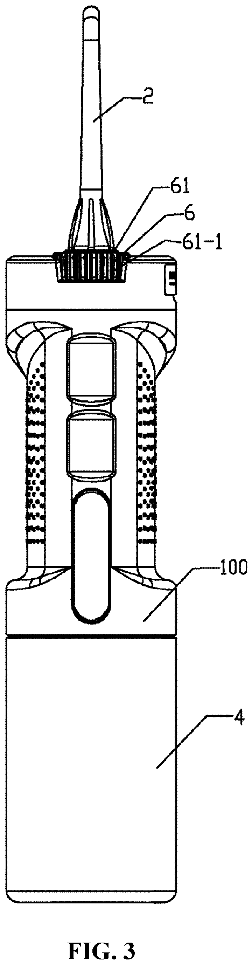

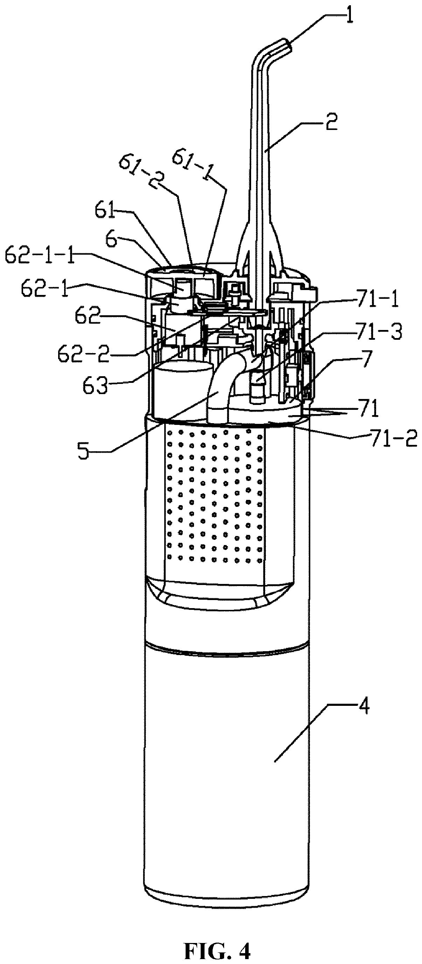

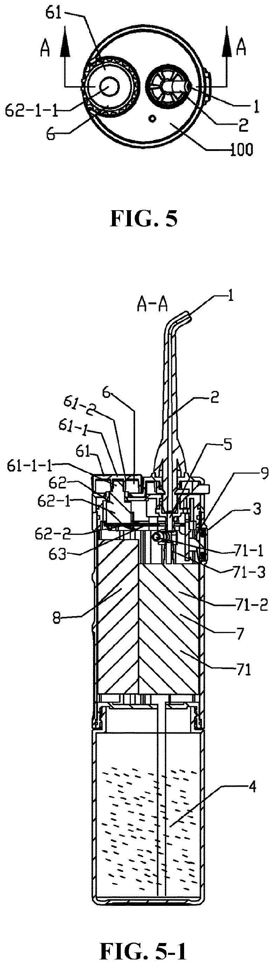

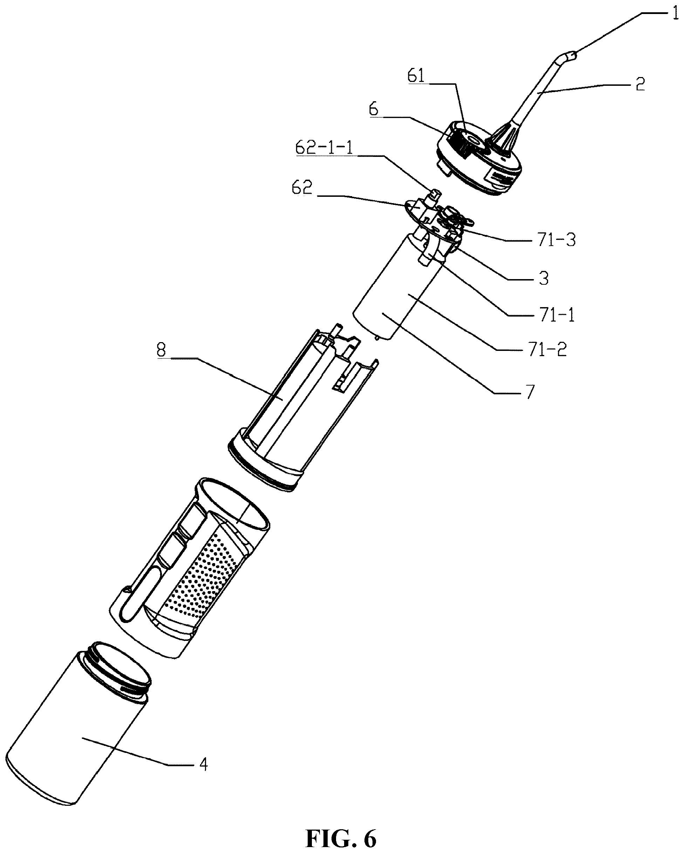

Referring to FIG. 1 to FIG. 6, an oral irrigator with a water volume stepless adjustment device of the present embodiment includes a sprayer 1, a spray lance 2, a control switch 3, a vessel 4, a water pipe 5, a water volume stepless adjustment device 6 and a water suction device 7.

The sprayer 1 is disposed at a front end of the spray lance 2. The control switch 3 is capable of controlling the spraying of a fluid in the spray lance 2. A fluid in the vessel 4 is connected to the spray lance 2 through the water pipe 5.

The water volume stepless adjustment device 6 includes an adjusting switch 61, a controller 62 and a signal output device 63. A signal output by the controller 62 can be continuously adjusted by adjusting the adjusting switch 61 so as to continuously adjust the magnitude of the pressure of the fluid sprayed from the spray lance 2.

In the present embodiment, the adjusting switch 61 is a knob type adjusting switch.

The adjusting switch 61 includes a knob 61-1 and a positioning hole 61-2. The controller 62 includes a potentiometer 62-1 and a control chip 62-2, an output signal of the potentiometer 62-1 is an input signal of the control chip 62-2, and an output signal of the control chip 62-2 can be controlled. The potentiometer 62-1 is provided with a connecting rod 62-1-1, and the connecting rod 62-1-1 can be embedded in the positioning hole 61-2. The knob 61-1 can be rotated to drive the connecting rod 62-1-1 to rotate to adjust the magnitude of the output signal of the potentiometer 62-1, thereby adjusting the output signal of the control chip 62-2.

In the present embodiment, the water suction device 7 is an electric water suction device 71. The electric water suction device 71 includes a water inlet pipe 71-1, a motor 71-2 and a water outlet pipe 71-3. The motor 71-2 works, the fluid in the vessel 4 is sucked into the electric water suction device 71 through the water pipe 5 and then sprayed from the water outlet pipe 71-3, and the sprayed fluid passes through the water pipe 5 and is then sprayed from the spray lance 2.

In the present embodiment, the knob 61-1 can be rotated to drive the connecting rod 62-1-1 to rotate to adjust an output voltage of the potentiometer 62-1, and the output voltage of the potentiometer 62-1 is an input voltage of the control chip 62-2. With the change of the input voltage, a pulse waveform output by the control chip 62-2 is also changed. A rotation speed of the motor 71-2 of the electric water suction device 71 can be adjusted according to the change of the pulse waveform to adjust a water suction speed of the electric water suction device 71, thereby achieving the purpose of adjusting the flow rate and pressure of the fluid sprayed from the spray lance 2.

The output signal of the potentiometer 62-1 may also be other electric signals, such as current, power, frequency or waveform. The output signal of the potentiometer 62-1 is the input signal of the control chip 62-2. The output signal of the control chip 62-2 may also be voltage, current, power, frequency or other control signals in addition to the pulse waveform signal described in the present embodiment, without departing from the scope of protection of the present application.

In the present embodiment, the rotation speed of the motor 71-2 is controlled by the output signal of the control chip 62-2 to adjust the water suction speed of the electric water suction device 71, thereby adjusting the flow rate and pressure of the fluid sprayed from the spray lance 2. Those skilled in the art can also design other control modes according to actual requirements, such as controlling the degree of vacuum of the water suction device 71 to control the water suction speed of the electric water suction device 71, without departing from the scope of protection of the present application.

In the present embodiment, the vessel 4 is detachably mounted at a proximal end of the oral irrigator 100. By virtue of a detachable connection mode, the vessel 4 can be conveniently detached and then cleaned, so that the vessel 4 is more sanitary and safe for long-term use.

The oral irrigator with a water volume stepless adjustment device of the invention also includes a power source 8 and a circuit system 9. The power source 8, the electric water suction device 71, the water volume stepless adjustment device 6 and the control switch 3 are connected together through the circuit system 9.

During use, the control switch 3 is turned on, the motor 71-2 of the electric water suction device 71 works, the fluid in the vessel 4 is sucked into the electric water suction device 71 through the water pipe 5 and then sprayed from the water outlet pipe 71-3, and the sprayed fluid enters the spray lance 2 through the water pipe 5 and is finally sprayed from the sprayer 1 of the spray lance 2. The knob 61-1 is rotated or the handle 61-3 is pushed back and forth to adjust the magnitude of the pressure of the sprayed fluid, an appropriate water pressure is selected according to personal preferences and cleaning effects, and then, the sprayer 1 is aligned to the part to be cleaned so as to clean the teeth and the oral cavity.

The knob type adjusting switch only needs to rotate the knob 61-1 back and forth to realize continuous adjustment of the signal of the controller 62, so that the use is simple and convenient.

The oral irrigator with a water volume stepless adjustment device of the present embodiment can continuously adjust the magnitude of the pressure of the fluid sprayed from the spray lance 2. When a user uses the oral irrigator with a water volume stepless adjustment device of the present embodiment, the user can adjust the water pressure according to personal tolerance and preferences until feel most comfortable, thereby greatly enhancing the cleaning effect and comfort of the oral irrigator in the using process.

### Example 2: slide groove adjusting type oral irrigator with water volume stepless adjustment device

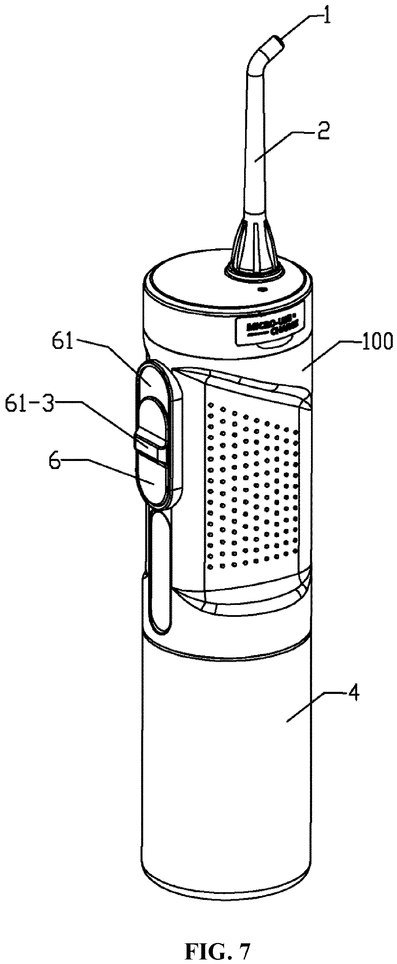

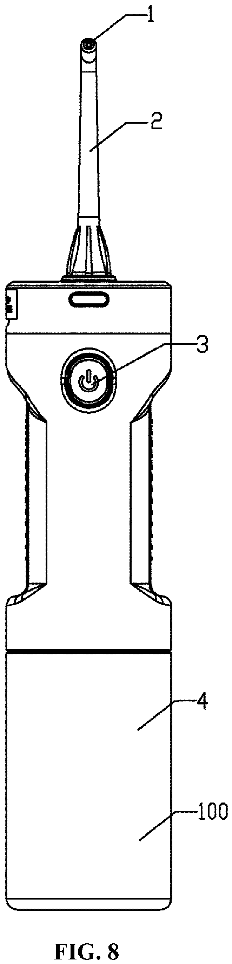

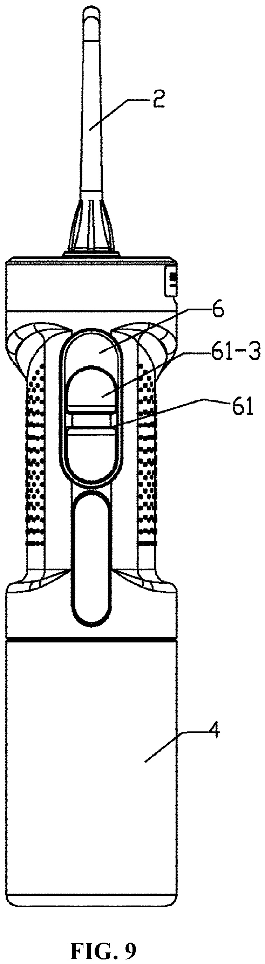

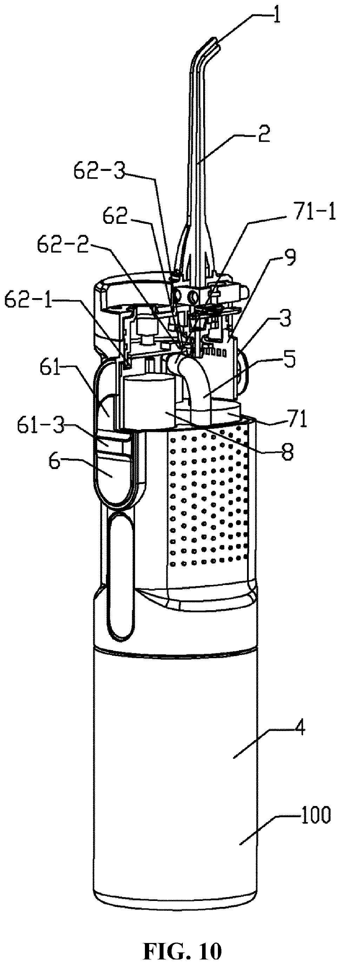

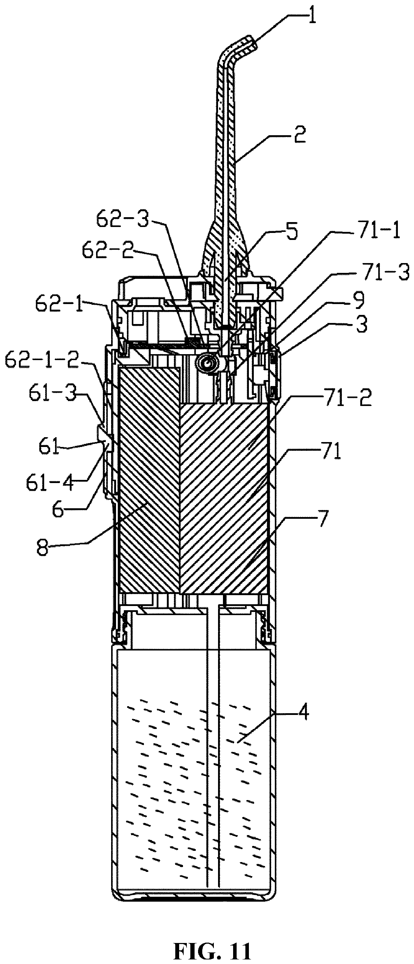

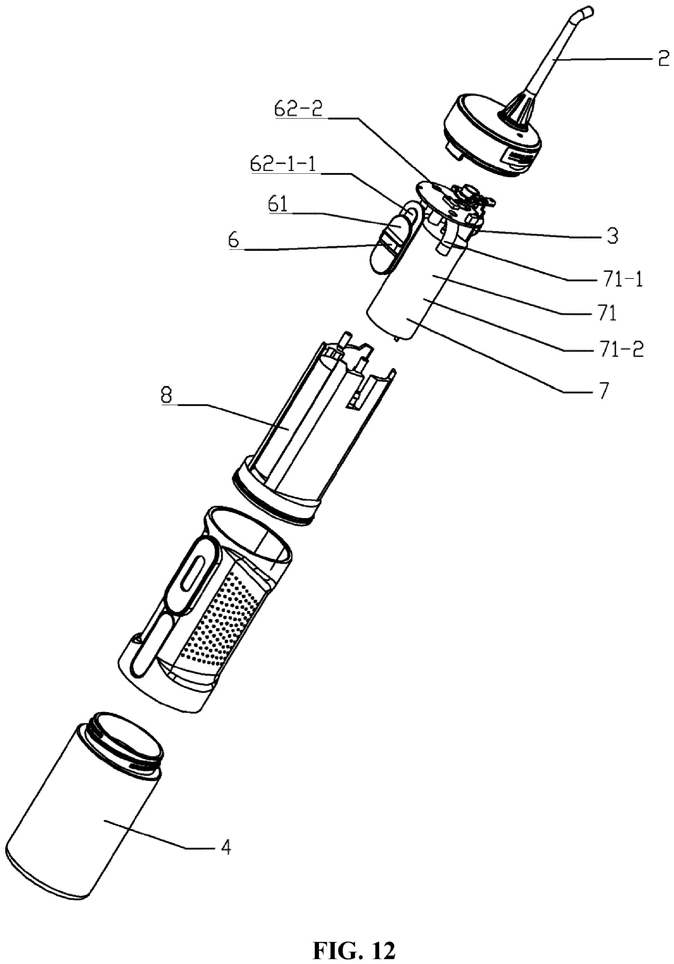

Referring to FIG. 7 to FIG. 12, the difference between the present example and Embodiment 1 lies in that in the present example, the adjusting switch 61-1 of the oral irrigator 100 is a slide groove type adjusting switch. The arrangement of the present example is outside the scope of the present invention but is included as useful for understanding the invention.

The adjusting switch 61 is a slide groove type adjusting switch.

In the present example, the adjusting switch 61 includes a handle 61-3 and a slide block 61-4, the potentiometer 62-1 is provided with a slide groove 62-1-2, and the slide block 61-4 is embedded in the slide groove 62-1-2.

The handle 61-3 is pushed back and forth to enable the slide block 61-4 to move back and forth in the slide groove 62-1-2 to adjust the magnitude of the output signal of the potentiometer 62-1, the output signal of the potentiometer 62-1 is the input signal of the control chip 62-2, and the output signal of the control chip 62-2 can be adjusted by adjusting the output signal of the control chip 62-2 to adjust the water suction speed of the electric water suction device 71, thereby achieving the purpose of adjusting the flow rate and pressure of the fluid sprayed from the spray lance 2.

By embedding the slide block 61-4 in the slide groove 62-1-2, signal adjustment of the controller 62 can be realized by just pushing the handle 61-3 back and forth, so that the operation is very convenient and smooth.

### Embodiment 3: oral irrigator with water volume stepless adjustment device of the invention, including pressure vessel

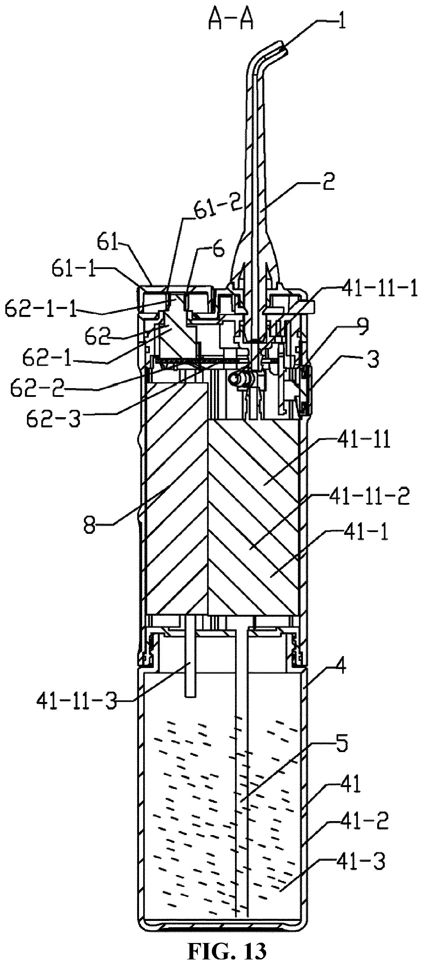

Referring to FIG. 13, the difference between the present embodiment and Embodiment 1 lies in that: in the present embodiment, the vessel 4 is a pressure vessel 41.

The pressure vessel 41 includes a pressurizing device 41-1, a housing 41-2 and a fluid containing space 41-3. The pressurizing device 41-1 capable of increasing the pressure of the fluid is mounted on the housing 41-2.

In the present embodiment, the pressurizing device 41-1 of the pressure vessel 41 is an electric pressurizing device 41-11. The electric pressurizing device 41-11 can increase the pressure of the fluid in the fluid containing space 41-3, so that the fluid in the fluid containing space 41-3 is pressed into the water pipe 5 from the fluid containing space 41-3 under the action of the pressure, and then is sprayed from the sprayer of the spray lance 2.

In the present embodiment, the electric pressurizing device 41-11 is an air compressor. The electric pressurizing device 41-11 includes an air inlet pipe 41-11-1, a compressor 41-11-2 and an air outlet pipe 41-11-3. During working, the compressor 41-11-2 operates, the air sucked from the air inlet pipe 41-11-1 is pressurized, and then, the fluid in the fluid containing space 41-3 is pressurized from the air outlet pipe 41-11-3 disposed above the fluid.

The controller 62 of the water volume stepless adjustment device 6 can control the magnitude of the pressure applied by the compressor 41-11-2 to the fluid in the fluid containing space 41-3 through the signal output by the output device 63, and the magnitude of the pressure of the fluid sprayed from the spray lance 2 can be controlled by continuously adjusting the magnitude of the applied pressure.

During use, the control switch 3 is turned on, the electric pressurizing device 41-11 works, the fluid in the fluid containing space 41-3 is pressurized, and under the action of the pressure, the fluid in the fluid containing space 41-3 is pressed into the spray lance 2 through the water pipe 5 and is finally sprayed from the sprayer. The knob 61-1 is rotated or the handle 61-3 is pushed back and forth to adjust the magnitude of the pressure of the sprayed fluid, an appropriate water pressure is selected according to personal preferences and cleaning effects, and then, the sprayer 1 is aligned to the part to be cleaned so as to clean the teeth and the oral cavity.

In the present embodiment, the electric pressurizing device 41-11 may also be pressurizing devices of other forms, such as a submersible pump. The fluid in the fluid containing space 41-3 is pressurized and then sprayed from the spray lance 2. Regardless of the form of the pressurizing device, it does not depart from the scope of protection of the present application.

### Embodiment 4: oral irrigator with water volume stepless adjustment device of the invention, connected to oral observation instrument

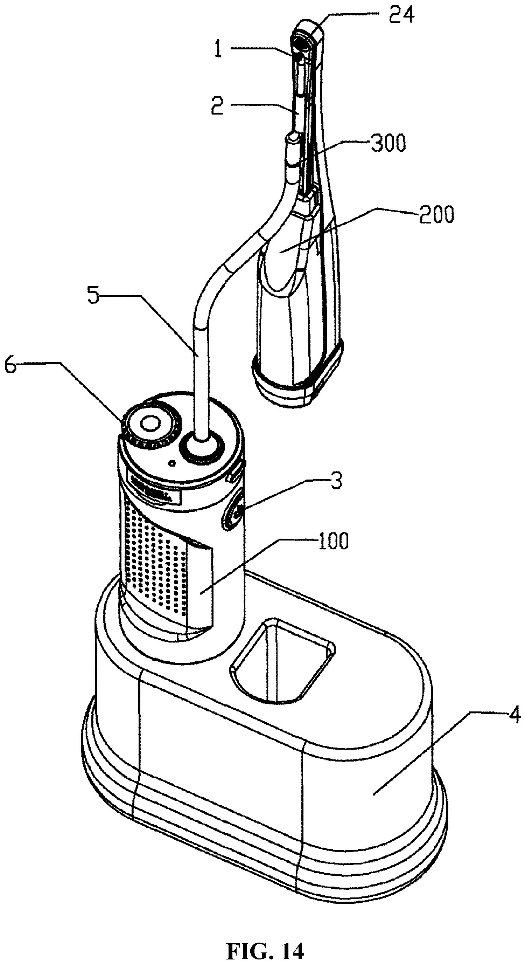

Referring to FIG. 14, the difference between the present embodiment and Embodiment 1 lies in that: in the present embodiment, the oral irrigator 100 with a water volume stepless adjustment device can be connected to the oral observation instrument 200 through the connecting mechanism 300 so as to clean the teeth and the oral cavity under direct vision.

During use, the observation system 24 of the oral observation instrument 200 is started, the control switch 3 is turned on, the motor 71-2 of the electric water suction device 71 works, the fluid in the vessel 4 is sucked into the electric water suction device 71 through the water pipe 5 and then sprayed from the water outlet pipe 71-3, and the sprayed fluid enters the spray lance 2 through the water pipe 5 and is finally sprayed from the sprayer 1 of the spray lance 2. After the pressure of the fluid is lowered, the sprayer 1 is aligned to the teeth or the interdental space needing to be cleaned, under direct vision, the adjusting switch 61 is adjusted to adjust the magnitude of the flow rate and speed of the fluid sprayed from the sprayer 1 until reaching a good cleaning effect and a high comfort degree, and then, the teeth and the oral cavity are cleaned under direct vision.

In the present embodiment, the sprayer 1 of the spray lance 2 is within the field of view of the observation system 24 of the oral observation instrument 200. Under the direct vision, the magnitude and speed of the fluid sprayed from the sprayer 1 can be observed in real time, thereby adjusting the water volume. Furthermore, under direct vision, the teeth and the oral cavity are cleaned, and the cleaning process can also be shot and photographed, so that the using process is safer and more reliable.

It should be noted that the structures disclosed and described herein may be replaced with other structures having the same effect, and the embodiments described herein are not the only structures that can implement the invention. Although the preferred embodiments of the invention have been described herein, it is apparent to those skilled in the art that the embodiments are merely examples, and various variations, modifications, and replacements may be made by those skilled in the art without departing from the invention. Therefore, the protection scope of the invention is as defined in the appended claims.

## Claims

1. A handheld oral irrigator (100), comprising a sprayer (1), a spray lance (2), a control switch (3), a vessel (4), a water pipe (5) and a water volume stepless adjustment device (6), all of which are disposed on the handheld oral irrigator (100), wherein:
the sprayer (1) is disposed at a front end of the spray lance (2);
the control switch (3) is capable of controlling the spraying of a fluid in the spray lance (2);
the vessel (4) is detachably mounted at a proximal end of the handheld oral irrigator (100); and a fluid in the vessel (4) is connected to the spray lance (2) through the water pipe (5);
the water volume stepless adjustment device (6) comprises:
a knob type adjusting switch (61);
a controller (62); and
a signal output device (63);
wherein a signal output by the controller (62) can be continuously adjusted by adjusting the adjusting switch (61) so as to continuously adjust the magnitude of pressure of the fluid sprayed from the spray lance (2);
wherein the adjusting switch (61) comprises a knob (61-1) and a positioning hole (61-2);
the controller (62) comprises a potentiometer (62-1) and a control chip (62-2), and an output signal of the control chip (62-2) can be controlled;
the potentiometer (62-1) is provided with a connecting rod (62-1-1), and the connected rod can be embedded in the positioning hole (61-2); the knob (61-1) can be rotated to drive the connecting rod (62-1-1) to rotate to adjust the magnitude of the output signal of the potentiometer (62-1), thereby adjusting the output signal of the control chip (62-2).

2. The oral irrigator according to claim 1, wherein the signal adjusted by the controller (62) is voltage, current, power, frequency, or waveform.

3. The oral irrigator according to claim 1, wherein the oral irrigator (100) comprises a water suction device (7) located on the oral irrigator (100); the water suction device (7) is capable of absorbing the fluid in the vessel (4) into the spray lance (2) through the water pipe (5), and then, the fluid is sprayed from the sprayer (1).

4. The oral irrigator according to claim 3, wherein the water suction device (7) is a manual water suction device or an electric water suction device (71).

5. The oral irrigator according to claim 4, wherein the water suction device (7) is an electric water suction device (71); the electric water suction device (71) comprises a water inlet pipe (71-1), a motor (71-2) and a water outlet pipe (71-3); the motor (71-2) works, the fluid in the vessel (4) is sucked into the electric water suction device (71) through the water pipe (5) and then sprayed from the water outlet pipe (71-3), and the sprayed fluid passes through the water pipe (5) and is then sprayed from the spray lance (2).

6. The oral irrigator according to claim 5, wherein after the output signal of the controller (62) is output by the signal output device (63), a rotation speed of the motor (71-2) can be controlled, and the rotation speed of the motor (71-2) can be adjusted by adjusting the output signal of the controller (62), thereby adjusting the magnitude of pressure of the fluid sprayed from the sprayer (1).

7. The oral irrigator according to claim 1, wherein the vessel (4) is a pressure vessel (41); the pressure vessel (41) comprises a pressurizing device (41-1), a housing (41-2) and a fluid containing space (41-3); the pressurizing device (41-1) capable of increasing the pressure of the fluid is mounted on the housing (41-2).

8. The oral irrigator according to claim 1, wherein the oral irrigator (100) can be connected to an oral observation instrument (200) through a connecting mechanism (300).

9. The oral irrigator according to claim 5, wherein the rotation speed of the motor (71-2) adjusts the water suction speed of the electric water suction device (71), thereby adjusting flow rate and pressure of the fluid sprayed from the spray lance (2).

10. The oral irrigator according to claim 7, wherein the controller (62) controls the magnitude of the pressure applied by the pressurizing device (41-1) to the fluid in the fluid containing space (41-3) through the signal output by the signal output device (63), and wherein the magnitude of the pressure of the fluid sprayed from the spray lance (2) is controllable by adjusting the magnitude of the applied pressure.

## Patentansprüche

1. Handgeführte Munddusche (100), umfassend eine Sprühvorrichtung (1), eine Sprühlanze (2), einen Steuerschalter (3), ein Gefäß (4), eine Wasserleitung (5) und eine Vorrichtung (6) zur stufenlosen Einstellung der Wassermenge, die alle an der handgeführten Munddusche (100) angeordnet sind, wobei:
die Sprühvorrichtung (1) an einem vorderen Ende der Sprühlanze (2) angeordnet ist;
der Steuerschalter (3) in der Lage ist, das Sprühen einer Flüssigkeit in der Sprühlanze (2) zu steuern;
der Behälter (4) abnehmbar an einem proximalen Ende der handgeführten Munddusche (100) angebracht ist; und eine Flüssigkeit in dem Behälter (4) über die Wasserleitung (5) mit der Sprühlanze (2) verbunden ist;
die Vorrichtung (6) zur stufenlosen Einstellung der Wassermenge umfasst:
einen Einstellschalter (61) in Form eines Knopfes;
eine Steuerung (62); und
eine Signalausgabevorrichtung (63);
wobei ein von der Steuerung (62) ausgegebenes Signal durch Einstellen des Einstellschalters (61) kontinuierlich eingestellt werden kann, um die Größe des Drucks des von der Sprühlanze (2) versprühten Fluids kontinuierlich einzustellen;
wobei der Einstellschalter (61) einen Knopf (61-1) und ein Positionierungsloch (61-2) umfasst;
die Steuerung (62) ein Potentiometer (62-1) und einen Steuerchip (62-2) umfasst und ein Ausgangssignal des Steuerchips (62-2) gesteuert werden kann;
das Potentiometer (62-1) mit einer Verbindungsstange (62-1-1) versehen ist und die Verbindungsstange in das Positionierungsloch (61-2) eingebettet werden kann; der Knopf (61-1) drehbar ist, um die Verbindungsstange (62-1-1) in Drehung zu versetzen, um die Größe des Ausgangssignals des Potentiometers (62-1) einzustellen, wodurch das Ausgangssignal des Steuerchips (62-2) eingestellt wird.

2. Munddusche nach Anspruch 1, bei der das durch die Steuereinheit (62) eingestellte Signal eine Spannung, ein Strom, eine Leistung, eine Frequenz oder eine Wellenform ist.

3. Munddusche nach Anspruch 1, wobei die Munddusche (100) eine Wasseransaugvorrichtung (7) umfasst, die sich an der Munddusche (100) befindet; die Wasseransaugvorrichtung (7) in der Lage ist, die Flüssigkeit im Behälter (4) durch die Wasserleitung (5) in die Sprühlanze (2) zu saugen, und die Flüssigkeit dann aus dem Sprühgerät (1) versprüht wird.

4. Munddusche nach Anspruch 3, bei der die Wasseransaugvorrichtung (7) eine manuelle Wasseransaugvorrichtung oder eine elektrische Wasseransaugvorrichtung (71) ist.

5. Munddusche nach Anspruch 4, bei der die Wasseransaugvorrichtung (7) eine elektrische Wasseransaugvorrichtung (71) ist; die elektrische Wasseransaugvorrichtung (71) ein Wasserzulaufrohr (71-1), einen Motor (71-2) und ein Wasserablaufrohr (71-3) umfasst; der Motor (71-2) arbeitet, die Flüssigkeit in dem Behälter (4) durch die Wasserleitung (5) in die elektrische Wassersaugvorrichtung (71) gesaugt und dann aus der Wasserauslassleitung (71-3) versprüht wird, und die versprühte Flüssigkeit die Wasserleitung (5) durchläuft und dann aus der Sprühlanze (2) versprüht wird.

6. Munddusche nach Anspruch 5, bei der, nachdem das Ausgangssignal der Steuerung (62) von der Signalausgabevorrichtung (63) ausgegeben wurde, eine Drehzahl des Motors (71-2) steuerbar ist und die Drehzahl des Motors (71-2) durch Einstellen des Ausgangssignals der Steuerung (62) einstellbar ist, wodurch die Druckstärke des aus dem Sprühgerät (1) gesprühten Fluids einstellbar ist.

7. Munddusche nach Anspruch 1, bei der das Gefäß (4) ein Druckgefäß (41) ist; das Druckgefäß (41) eine Druckvorrichtung (41-1), ein Gehäuse (41-2) und einen Flüssigkeit enthaltenden Raum (41-3) umfasst; und die Druckvorrichtung (41-1), die in der Lage ist, den Druck der Flüssigkeit zu erhöhen, an dem Gehäuse (41-2) angebracht ist.

8. Munddusche nach Anspruch 1, wobei die Munddusche (100) über einen Verbindungsmechanismus (300) mit einem Mundbeobachtungsinstrument (200) verbindbar ist.

9. Munddusche nach Anspruch 5, bei der die Rotationsgeschwindigkeit des Motors (71-2) die Wasseransauggeschwindigkeit der elektrischen Wasseransaugvorrichtung (71) reguliert, wodurch die Durchflussrate und der Druck der aus der Sprühlanze (2) versprühten Flüssigkeit reguliert werden.

10. Munddusche nach Anspruch 7, bei der die Steuerung (62) die Größe des Drucks, der durch die Druckvorrichtung (41-1) auf die Flüssigkeit in dem Flüssigkeit enthaltenden Raum (41-3) ausgeübt wird, durch das von der Signalausgabevorrichtung (63) ausgegebene Signal steuert, und
bei der die Größe des Drucks der von der Sprühlanze (2) versprühten Flüssigkeit durch Einstellen der Größe des ausgeübten Drucks steuerbar ist.

## Revendications

1. Irrigateur buccal à main (100), comprenant un pulvérisateur (1), une pointe jet (2), un commutateur de commande (3), un contenant (4), un tuyau d'eau (5) et un dispositif de réglage progressif de volume d'eau (6), chacun d'eux est disposé sur l'irrigateur buccal à main (100), dans lequel :
le pulvérisateur (1) est disposé au niveau d'une extrémité avant de la pointe jet (2) ;
le commutateur de commande (3) est apte à commander la pulvérisation d'un fluide dans la pointe jet (2) ;
le contenant (4) est monté de manière détachable au niveau d'une extrémité proximale de l'irrigateur buccal à main (100) ; et un fluide dans le contenant (4) est raccordé à la pointe jet (2) par l'intermédiaire du tuyau d'eau (5) ;
le dispositif de réglage progressif de volume d'eau (6) comprend :
un commutateur de réglage de type molette (61) ;
un dispositif de commande (62) ; et
un dispositif de sortie de signal (63) ;
dans lequel un signal délivré par le dispositif de commande (62) peut être réglé en continu par réglage du commutateur de réglage (61) de manière à régler en continu l'amplitude d'une pression du fluide pulvérisé par la pointe jet (2) ;
dans lequel le commutateur de réglage (61) comprend une molette (61-1) et un trou de positionnement (61-2) ;
le dispositif de commande (62) comprend un potentiomètre (62-1) et une puce de commande (62-2), et un signal de sortie de la puce de commande (62-2) peut être commandé ;
le potentiomètre (62-1) est doté d'une tige de liaison (62-1-1), et la tige liée peut être intégrée dans le trou de positionnement (61-2) ; la molette (61-1) peut être tournée pour amener la tige de liaison (62-1-1) à tourner pour régler l'amplitude du signal de sortie du potentiomètre (62-1), réglant ainsi le signal de sortie de la puce de commande (62-2).

2. Irrigateur buccal selon la revendication 1, dans lequel le signal réglé par le dispositif de commande (62) est la tension, le courant, la puissance, la fréquence, ou la forme d'onde.

3. Irrigateur buccal selon la revendication 1, dans lequel l'irrigateur buccal (100) comprend un dispositif d'aspiration d'eau (7) situé sur l'irrigateur buccal (100) ; le dispositif d'aspiration d'eau (7) est apte à absorber le fluide dans le contenant (4) dans la pointe jet (2) à travers le tuyau d'eau (5), puis le fluide est pulvérisé par le pulvérisateur (1).

4. Irrigateur buccal selon la revendication 3, dans lequel le dispositif d'aspiration d'eau (7) est un dispositif d'aspiration d'eau manuel ou un dispositif d'aspiration d'eau électrique (71).

5. Irrigateur buccal selon la revendication 4, dans lequel le dispositif d'aspiration d'eau (7) est un dispositif d'aspiration d'eau électrique (71) ; le dispositif d'aspiration d'eau électrique (71) comprend un tuyau d'entrée d'eau (71-1), un moteur (71-2) et un tuyau de sortie d'eau (71-3) ; le moteur (71-2) en fonctionnement, le fluide dans le contenant (4) est aspiré dans le dispositif d'aspiration d'eau électrique (71) à travers le tuyau d'eau (5), puis est pulvérisé à partir du tuyau de sortie d'eau (71-3), et le fluide pulvérisé passe à travers le tuyau d'eau (5), puis est pulvérisé par la pointe jet (2).

6. Irrigateur buccal selon la revendication 5, dans lequel une fois le signal de sortie du dispositif de commande (62) délivré par le dispositif de sortie de signal (63), une vitesse de rotation du moteur (71-2) peut être commandée, et la vitesse de rotation du moteur (71-2) peut être réglée par réglage du signal de sortie du dispositif de commande (62), réglant ainsi l'amplitude de pression du fluide pulvérisé par le pulvérisateur (1).

7. Irrigateur buccal selon la revendication 1, dans lequel le contenant (4) est un contenant sous pression (41) ; le contenant sous pression (41) comprend un dispositif de mise sous pression (41-1), un boîtier (41-2) et un espace contenant du fluide (41-3) ; le dispositif de mise sous pression (41-1) apte à augmenter la pression du fluide est monté sur le boîtier (41-2).

8. Irrigateur buccal selon la revendication 1, dans lequel l'irrigateur buccal (100) peut être raccordé à un instrument d'observation buccale (200) par l'intermédiaire d'un mécanisme de liaison (300).

9. Irrigateur buccal selon la revendication 5, dans lequel la vitesse de rotation du moteur (71-2) règle la vitesse d'aspiration d'eau du dispositif d'aspiration d'eau électrique (71), réglant ainsi le débit et la pression du fluide pulvérisé par la pointe jet (2).

10. Irrigateur buccal selon la revendication 7, dans lequel le dispositif de commande (62) commande l'amplitude de la pression appliquée par le dispositif de mise sous pression (41-1) sur le fluide dans l'espace contenant du fluide (41-3) par l'intermédiaire du signal délivré par le dispositif de sortie de signal (63), et dans lequel l'amplitude de la pression du fluide pulvérisé par la pointe jet (2) est commandable par réglage de l'amplitude de la pression appliquée.
